# EUROPEAN PATENT SPECIFICATION

(11) **EP 0 458 414 B1**
(45) Date of publication and mention of the grant of the patent: **11.09.1996**
(21) Application number: 91201201.0
(22) Date of filing: 17.05.1991
(51) Int. Cl.: C01B 39/00, C07C 2/12, B01J 29/06

(54) **Metals-containing zeolites, a process for preparing such zeolites and their use in catalytic processes**
Metallhaltige Zeolithe, Verfahren zu ihrer Herstellung und ihre Verwendung in katalytischen Prozessen
Zéolites contenant des métaux, leur procédé de préparation et leur utilisation dans des procédés catalytiques

(30) Priority: 22.05.1990 GB 9011411
(43) Date of publication of application: 27.11.1991
(73) Proprietor: SHELL INTERNATIONALE RESEARCH MAATSCHAPPIJ B.V., NL-2596 HR Den Haag (NL)
(72) Inventor: Post, Martin Franciscus Maria, NL-1031 CM Amsterdam (NL); Wielers, Antonius Franziscus Heinrich, NL-1031 CM Amsterdam (NL)

(56) References cited:
- FR-A- 2 200 048
- US-A- 3 576 901
- US-A- 4 344 868
- P. J. GROBET, W. J. MORTIER, E. F. VANSANT AND G. SCHULZ-EKLOFF EDITORS 'INNOVATION IN ZEOLITE MATERIALS SCIENCE. PROCEEDINGS OF AN INTERNATIONAL SYMPOSIUM, NIEUWPORT, BELGIUM, SEPTEMBER 13-17, 1987' , ELSEVIER SCIENCE PUBLISHERS B. V., AMSTERDAM B. WICHTERLOVA AND AL. " SOLID-STATE INTERACTIONS OF Mn OR Fe CATIONS WITH ZSM-5 ZEOLITES " . PAGES 199-206
- H. K. KARGE, J. WEITKAMP EDITORS 'ZEOLITES AS CATALYSTS, SORBENTS AND DETERGENT BUILDERS. PROCEEDINGS OF AN INTERNATIONAL SYMPOSIUM, WÜRSBURG,F.R.G., SEPTEMBER 4-8, 1988' , ELSEVIER SCIENCE PUBLISHERS B. V. , AMSTERDAM B. WICHTERLOVA AND AL. " PREPARATION OF NiHZSM-5 CATALYST FOR ISOMERISATION OF C8 AROMATICS. SOLID-STATE INCORPORATION OF NICKEL ". PAGES 347-353
- WORLD PATENTS INDEX LATEST Week 8527, Derwent Publications Ltd., London, GB; AN 85-163905 & SU-A-1 130399

## Description

The present invention relates to metals-containing zeolites, a process for preparing such zeolites and their use in catalytic processes.

It is well known in the art that zeolites can be modified in many ways depending on the applications envisaged. Convenient as well as conventional ways of modifying zeolites comprise ion-exchange with aqueous solutions of the appropriate metal compounds.

It is also known that the use of aqueous solutions of metal compounds has limitations with respect to the amount of metal ions which can be exchanged. Also the Si/Al atomic ratio of the zeolite plays a role in the amount of metals to be incorporated. When the content of alumina in the framework of a zeolite is reduced the amount of metal to be incorporated is also reduced substantially. This has of course a negative effect for many reactions wherein the overall loading of the metal is a very important parameter in the overall activity, such as for instance the oligomerisation of lower olefins.

Apart from activity considerations, the selectivity attainable plays a very important role in catalysed reactions. In reactions wherein zeolites are used as catalysts or catalyst carriers the level of acidity is a factor which has to be taken into account from a selectivity point of view. In the event that the intrinsic acidity of a zeolite, referred to hereinafter as Bronsted acidity, is a property which does not contribute or even affects the particular reaction envisaged it would be useful to be able to curb the influence caused by the Bronsted acidity.

Zeolites having a reduced Bronsted acidity, in particular a very low residual Bronsted acidity, containing also one or more metal compounds having intrinsic catalytic activity, in particular when a relative high amount of catalytically active metal(s) is present, would be of great interest.

The present invention relates to zeolites comprising a metal function A and a metal function B wherein function A comprises an alkali metal moiety and function B comprises at least one metal moiety of a metal of Group VIB, VIIB or VIII, which zeolites have a Si/Al atomic ratio of at least 3 and a A/Aₒ value (as defined hereinafter) of at most 0.05 whilst the alkali/aluminium atomic ratio is between 0.6 and 1.1.

The zeolites according to the present invention comprise two metal functions (defined as A and B) and have a very low residual Bronsted acidity. The maximum level of Bronsted acidity present in a zeolite in accordance with the present invention is defined by the expression "A/Aₒ at most 0.05". In said expression Aₒ represents the absorbance of the OH-band, having its peak maximum in the frequency range between 3600 cm⁻¹ and 3650 cm⁻¹ (depending on the nature of the zeolite) in the Infrared spectrum of the corresponding zeolite sample in the H-form (i.e. not containing metal function A) and A the absorbance of the OH-band at the corresponding wave number of the zeolite according to the present invention. In other words, the zeolites according to the present invention possess at most 5% of the relative absorbance of the OH- groups present when the zeolite would be in the corresponding H-form (in the absence of metal function A). Preferably, the value A/Aₒ is below 0.03, in particular below 0.02.

The metal function A comprises an alkali metal moiety. Preference is given to potassium. It is possible, though not preferred, to have mixtures of alkali metal moieties present such as a mixture of potassium and sodium. It is also possible to have one or more alkaline earth metal moieties present as well, such as barium or calcium.

The metal function B comprises one or more metal moieties of a metal of Group VIB, VIIB or VIII. Examples of Group VIB metals are chromium, molybdenum and tungsten. Manganese is an example of a Group VIIB metal. Examples of Group VIII metals are cobalt, nickel, platinum and palladium. If desired, two or more metal functions B can be present in the metals-containing zeolites. It is also possible that in addition to the metal function B one or more other metal compounds of Group IB, IIB, IIIA and IVA are present in the zeolites according to the present invention. Preferably, the zeolites comprise a metal function B comprising one metal moiety. In particular, the metal function B comprises a Group VIII metal moiety. Suitably, the Group VIII metal moiety comprises nickel.

The zeolites according to the present invention although containing at least two different types of metal (A and B) can be considered from a catalytic point of view as operating monofunctionally. They have a Si/Al atomic ratio of of least 3. Preference is given to zeolites having a Si/Al atomic ratio between 5 and 100. Suitably the zeolites contain ring structures characterised by containing up to 12 T-atoms (tetrahedrally coordinated atoms), suitably Si + Al atoms. Preferably, the zeolites according to the present invention contain ring structures containing up to 10 Si + Al atoms. Zeolite structures referred to as MFI, MEL and FER suitably serve as backbone for the metals-containing zeolites according to the present invention. Examples of such zeolites are ZSM-5, ZSM-11, US-Y, ferrierite and mordenite.

The zeolites according to the present invention have an alkali/aluminium atomic ratio between 0.6 and 1.1. Preference is given to zeolites having an alkali/aluminium atomic ratio between 0.65 and 1. Since the zeolites according to the present invention have an alkali/aluminium atomic ratio of at least 0.60 it is believed that the intrinsic Bronsted acidity has been neutralised to such an extent (expressed in terms of A/Aₒ) that the catalytic activity is the dominating property. Therefore, the zeolites according to the present invention are defined as monofunctional despite the fact that they contain two different metal functions.

It has been found that the zeolites according to the present invention can be suitably prepared by subjecting a zeolite having a Si/Al atomic ratio of at least 3 and an alkali/aluminium atomic ratio between 0.6 and 1.1 together with a Group VIB, VIIB or VIII metal compound to solid state exchange conditions including a heat treatment to reduce the A/Aₒ value to at most 0.05.

Solid state exchange conditions as defined for the purpose of the present invention comprise preparing physical mixtures of zeolites (having a Si/Al atomic ratio of at least 3 and an alkali/aluminium ratio between 0.6 and 1.1) and Group VIB, VIIB or VIII metal compounds, which mixtures may be subjected to one or more shaping treatments such as pelletising and which are subjected to a heat treatment to substantially effect the solid state exchange reaction, thus giving monofunctional zeolites comprising two metal functions.

The process according to the present invention is suitably carried out by mixing a Group VIB, VIIB or VIII metal compound with the zeolite as defined hereinbefore. Preference is given to the use of Group VIII metal compounds, in particular nickel compounds.

It has been found that metal oxides as well as metal salts, preferably in the form of the corresponding hydrates can be suitably applied in the solid state exchange reaction according to the present invention. In particular salts containing decomposable anions such as nitrates and nitrites can be used advantageously in the preparation of the zeolites according to the present invention. A particularly preferred compound is nickel nitrate nonahydrate.

After mixing and preferably pelletising the starting materials the physical mixture, preferably in a shaped form is subjected to a heat treatment to produce the zeolites having a A/Aₒ value of at most 0.05 as defined hereinbefore.

The heat treatment is suitably carried out at a temperature between 300 °C and 800 °C. Preference is given to a heat treatment at a temperature between 350 °C and 600 °C. The heat treatment is suitably carried out in an oxidative environment which facilitates the preparation of the (monofunctional) zeolites according to the present invention. The heat treatment is suitably carried out using air, optionally diluted with nitrogen. It is also possible to use oxygen, if desired diluted with an inert gas such as helium or argon as the environment to perform the heat treatment.

The zeolites which are used as starting materials in the process for preparing the zeolites according to the present invention, i.e. the zeolites having an Si/Al atomic ratio of at least 3 and an alkali/aluminium atomic ratio between 0.6 and 1.1, can be prepared themselves by treating the corresponding H⁺- and/or NH₄⁺- zeolites having a Si/Al atomic ratio of at least 3 with the appropriate alkali metal compounds. Suitably, by ion-exchanging the hydrogen and/or ammonium ions with an alkali ion, i.e. by treatment with an aqueous solution of the appropriate alkali metal salt of the desired concentration the zeolites having an alkali/aluminium atomic ratio between 0.6 and 1.1. can be prepared. Advantageously, zeolites having a Si/Al atomic ratio of at least 3 are treated with an aqueous solution of a potassium salt of sufficient concentration to produce a starting material for the preparation of the zeolites according to the present invention.

It should be observed that the technique to incorporate metal ions into conventional zeolites by solid state exchange is known per se. Reference is made in this respect to the article by B. Wichterlova et al. (in "Zeolites as Catalysts, Sorbents and Detergent Builders", Editors H.G.Karge and J. Weitkamp, issued by Elseviers Science Publishers B.V., Amsterdam 1989 pages 347 - 353) wherein the preparation of NiHZSM-5 catalysts for the isomerisation of C₈ aromatics is described. The introduction of Na⁺ in conventional zeolites (i.e. zeolites having a low Si/Al atomic ratio) such as Ca-Y, Ba-Y and La-Y by calcining mixtures of the respective zeolites with a sodium salt has been described by J.A. Rabo and P.H. Kasai (Progr. in Solid State Chemistry 9 (1975) 1).

The zeolites can be used as such as well as together with a binder. Suitable binders comprise alumina, silica or silica-alumina. Normally, the zeolites, optionally together with a binder, will be used in the form of extrudates, e.g. in the form of cylindrical or polylobal extrudates. The preparation of catalysts containing both a zeolite in accordance with the present invention and a binder can be carried out by conventional routes such as mulling, pelletising or extruding.

The zeolites according to the present invention can be used as such or together with a binder in catalytic reactions, in particular in catalytic reactions which favour the absence of acidic sites.

It has been found that the zeolites according to the present invention can be used advantageously in the oligomerisation of alkenes. In particular a high selectivity to linear oligomers has been observed which is attributed to the substantial absence of acidic sites in the zeolitic catalysts used in the oligomerisation reaction. Preferably, alkenes containing up to 6 carbon atoms are used as feedstock in the oligomerisation process according to the present invention. Good results have been obtained using ethylene and 1-butene, in particular ethylene, as feedstock in the oligomerisation reaction.

The oligomerisation reaction is suitably carried out at a temperature between 150 °C and 400 °C, in particular between 200 °C and 350 °C. The oligomerisation reaction can be carried out at autogenic pressure or above, e.g. up to 100 bar. Suitably, the oligomerisation reaction is carried out at a pressure between 10 and 50 bar.

If desired, the oligomers obtained can be subjected to further treatment depending on the intended use of the products obtained. Suitably, the oligomers can be subjected to a distillation treatment to isolate fractions which are to be treated or used separately.

The invention will now be elucidated with the following Examples.

### EXAMPLE I

### Catalyst preparation

The starting materials were brought firstly in the K-form by exchange with a 1 M KNO3 solution. For the solid state ion exchange procedure intimate physical mixtures of the zeolite in the K-form and the desired nickel salt (nickel nitrate (Ni(NO₃)₂.9H₂O, ex Merck) were prepared by thoroughly grinding the desired amounts of the two components in a mortar. About 2 grams of zeolite was used and the amount of nickel nitrate mixed with the zeolite was adjusted in order to arrive at a substoichiometric Ni/Al (mol.%/ mol.%) ratio of about 0.4. Thereafter the mixture was pelletised and crushed to obtain a 30-80 mesh fraction. Subsequently the physical mixture was heated in a He stream at 130 °C for about 4 hrs and finally the temperature was increased to 520 °C. The sample was kept at this temperature for at least 2 h. After cooling down to room temperature the catalyst is ready for use in the reactor. Some data are given in Table 1 (VUS-Y : very ultra-stable zeolite Y, MOR : mordenite and ERI : erionite).

**TABLE 1**

| Base Material | Si/Al (mol/mol) | Ni (%w) | Ni/Al (mol/mol) |
|---|---|---|---|
| VUS-Y | 10.5 | 2.16 | 0.265 |
| MOR | 10 | 2.16 | 0.242 |
| ZSM-11 | 21 | 2.16 | 0.500 |
| ERI | 4 | 2.16 | 0.097 |

### EXAMPLE II

### Oligomerisation of ethylene and 1-butene

The series of catalysts prepared as described in Example I was used as catalysts for the conversion of 1-butene or ethylene. The catalysts were loaded in a micro-flow reactor . Subsequently the catalysts were treated for 1 h in He stream at 450 °C. Thereafter the catalysts were cooled down to the desired reactor temperature (see below). At this temperature the reactor was pressurized with Helium and subsequently either ethylene or 1-butene was introduced. Experimental conditions are given below and results are collected in the Tables 2 and 3.

### Conditions

| ethylene experiments | 1-butene experiments |
|---|---|
| Temperature = 220 °C | Temperature = 220 °C |
| Total pressure= 30 bar | Total pressure = 20 bar |
| molar He/C₂⁼ = 2 mol/mol | Molar He/1-C₄⁼ = 3 mol/mol |
| WHSV (ethylene) = 2 g/(g.h) | WHSV (1-C₄⁼) = 2 g/(g.h) |

**TABLE 2**

| PERFORMANCE (at runhour 5) OF VARIOUS CATALYSTS IN THE OLIGOMERISATION OF ETHYLENE | | | | | | | | | |
|---|---|---|---|---|---|---|---|---|---|
| Catalysts | Conv. | C4* | C5 | C6 | linC6 | C7 | C8 | linC8 | >C8 |
| Ni,K-ERI | 14.98 | 85.41 | - | 11.68 | 65.60 | - | 2.33 | 51.27 | 0.27 |
| Ni,K-ZSM11 | 30.51 | 83.26 | 0.98 | 5.80 | 81.80 | - | 2.72 | 50.28 | 7.30 |
| Ni,K-MOR | 14.98 | 87.63 | - | 10.29 | 67.54 | - | 2.08 | 47.32 | 0.0 |
| Ni,K-VUSY | 8.69 | 82.70 | 3.16 | 9.88 | 44.36 | - | 2.02 | - | 2.2 |

| | | | | | | | | | |
|---|---|---|---|---|---|---|---|---|---|
| * Selectivities are expressed in the fraction ethylene converted to a particular product. Selectivity for the formation of linear products is expressed as the fraction linear hydrocarbons in a particular hydrocarbon fraction. | | | | | | | | | |

**TABLE 3**

| YIELDS (at runhour 5 h) IN THE OLIGOMERISATION OF 1-BUTENE | | |
|---|---|---|
| Catalysts | Yield C₈ | fraction linear C₈ |
| Ni,K-ERI | 2.8 | 28 |
| Ni,K-ZSM11 | 5.5 | 62 |
| Ni,K-MOR | 2.1 | 37 |
| Ni,K-VUSY | 2.4 | 6 |

| | | |
|---|---|---|
| * Selectivities to the various hydrocarbons are expressed in the fraction off 1-butene converted to a particular product. Selectivities to linear product is the fraction (as determined with the on-line GC) of linear products present in a particular hydrocarbon fraction. | | |

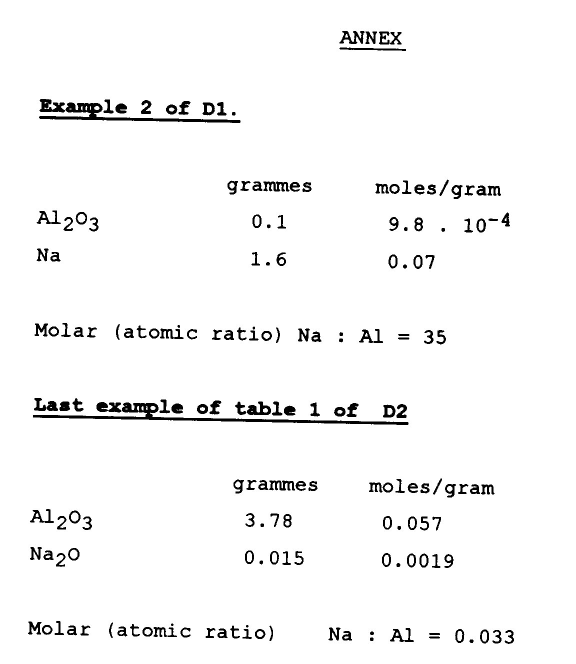

## Claims

1. Zeolites comprising a metal function A and a metal function B wherein function A comprises an alkali metal moiety and function B comprises at least one metal moiety of a metal of Group VIB, VIIB or VIII, which zeolites have a Si/Al atomic ratio of at least 3 and an A/Aₒ value of at most 0.05 whilst the alkali/aluminium atomic ratio is between 0.6 and 1.1, wherein Aₒ represents the absorbance of the OH-band, having its peak maximum in the frequency range between 3600 cm⁻¹ and 3650 cm⁻¹ in the Infrared spectrum of the corresponding zeolite sample in the H-form and wherein A represents the absorbance of the OH-band at the corresponding wave number of the zeolite according to the present invention.

2. Zeolites according to claim 1, wherein the function A comprises a potassium moiety.

3. Zeolites according to claim 1 or 2, wherein the function B comprises a Group VIII metal moiety, in particular Ni.

4. Zeolites according to one or more of claims 1 - 3, wherein the Si/Al atomic ratio is between 5 and 100.

5. Zeolites according to one or more of claims 1 - 4, wherein the zeolites have ring structures containing up to 12 T- atoms, in particular of the MFI or MEL type.

6. Zeolites according to one or more of claims 1 - 5, wherein the alkali/aluminium atomic ratio is between 0.65 and 1.

7. Process for preparing zeolites according to one or more of claims 1-6 comprising subjecting a zeolite having an Si/Al atomic ratio of at least 3 and an alkali/aluminium atomic ratio between 0.6 and 1.1 together with a Group VIB, VIIB or VIII metal compound to solid state exchange conditions including a heat treatment to reduce the A/Aₒ value to at most 0.05, wherein Aₒ represents the absorbance of the OH-band, having its peak maximum in the frequency range between 3600 cm⁻¹ and 3650 cm⁻¹ in the Infrared spectrum of the corresponding zeolite sample in the H-form and wherein A represents the absorbance of the OH-band at the corresponding wave number of the zeolite according to the present invention.

8. Process according to claim 7, comprising subjecting the zeolite to a treatment with a Group VIII metal compound, in particular with a nickel compound.

9. Process according to claim 7 or 8, comprising subjecting the zeolite to a treatment with a metal compound containing a decomposable anion, in particular with a nitrate.

10. Process according to claim 9, comprising subjecting the zeolite to a treatment with nickel nitrate nonahydrate.

11. Process according to one or more of claims 7 - 10, comprising a pelletising treatment prior to the heat treatment.

12. Process according to one or more of claims 7 - 11 wherein the heat treatment is carried out at a temperature between 300 °C and 800 °C.

13. Process according to one or more of claims 7 - 12 wherein the heat treatment is carried out in an oxidative environment.

14. Process for the oligomerisation of alkenes comprising reacting one or more alkenes with one or more zeolites according to one or more of claims 1 - 6 and recovering oligomerised products therefrom.

15. Process according to claim 14 comprising using one or more alkenes having up to 6 carbon atoms as feedstock to produce oligomers, in particular using ethylene or 1-butene as feedstock.

16. Process according to claim 14 or 15 wherein the reaction is carried out at a temperature between 150 °C and 400 °C and at a pressure up to 100 bar, in particular at a temperature between 200 °C and 350 °C and at a pressure between 10 and 50 bar.

17. Catalysts suitable for use in a process as claimed in one or more of claims 14 - 16 comprising a zeolite as claimed in one or more of claims 1 - 6 and a binder.

18. Catalysts according to claim 17 comprising a zeolite prepared in accordance with a process as claimed in one or more of claims 7 - 13.

19. Oligomerised products whenever obtained by a process as claimed in one or more of claims 14 - 16.

## Patentansprüche

1. Zeolithe mit einem Gehalt an einer Metallfunkiton A und einer Metallfunktion B, worin die Funktion A einen Alkalimetallrest und die Funktion B wenigstens einen Metallrest eines Metalles der Gruppe VIB, VIIB oder VIII umfaßt, welche Zeolithe ein Si/Al-Atomverhältnis von wenigstens 3 und einen A/Aₒ-Wert von höchstens 0,05 aufweisen, während das Alkali/Aluminium-Atomverhältnis zwischen 0,6 und 1,1 liegt, worin Aₒ die Absorption der OH-Bande, die ihr Peak-Maximum im Frequenzbereich zwischen 3600 cm⁻¹ und 3650 cm⁻¹ im Infrarotspektrum der entsprechenden Zeolithprobe in der H-Form hat, und A die Absorption der OH-Bande an der entsprechenden Wellenzahl des Zeolithen gemäß der vorliegenden Erfindung darstellt.

2. Zeolithe nach Anspruch 1, worin die Funktion A einen Kaliumrest umfaßt.

3. Zeolithe nach Anspruch 1 oder 2, worin die Funktion B einen Gruppe VIII-Metallrest umfaßt, insbesondere Ni.

4. Zeolithe nach einem oder mehreren der Ansprüche 1 bis 3, worin das Si/Al-Atomverhältnis zwischen 5 und 100 beträgt.

5. Zeolithe nach einem oder mehreren der Ansprüche 1 bis 4, worin die Zeolithe Ringstrukturen mit einem Gehalt an bis zu 12 T-Atomen aufweisen, insbesondere vom MFI- oder MEL-Typ.

6. Zeolithe nach einem oder mehreren der Ansprüche 1 bis 5, worin das Alkali/Aluminium-Atomverhältnis zwischen 0,65 und 1 beträgt.

7. Verfahren zur Herstellung von Zeolithen nach einem oder mehreren der Ansprüche 1 bis 6, umfassend das Aussetzen eines Zeolithen mit einem Si/Al-Atomverhältnis von wenigstens 3 und einem Alkali/Aluminium-Atomverhältnis zwischen 0,6 und 1,1, zusammen mit einer Gruppe VIB-, VIIB- oder VIII-Metallverbindung, Festphasenaustauschbedingungen, einschließlich einer Wärmebehandlung, zur Verringerung des A/Aₒ-Wertes auf höchstens 0,05, worin Aₒ die Absorption der OH-Bande, die ihr Peak-Maximum im Frequenzbereich zwischen 3600 cm⁻¹ und 3650 cm ⁻¹ im Infrarotspektrum der entsprechenden Zeolithprobe in der H-Form hat, und A die Absorption der OH-Bande an der entsprechenden Wellenzahl des Zeolithen gemäß der vorliegenden Erfindung darstellt.

8. Verfahren nach Anspruch 7, umfassend das Aussetzen des Zeolithen einer Behandlung mit einer Gruppe VIII-Metallverbindung, insbesondere mit einer Nickelverbindung.

9. Verfahren nach Anspruch 7 oder 8, umfassend das Aussetzen des Zeolithen einer Behandlung mit einer Metallverbindung, die ein zersetzliches Anion enthält, insbesondere mit einem Nitrat.

10. Verfahren nach Anspruch 9, umfassend das Aussetzen des Zeolithen einer Behandlung mit Nickelnitratnonahydrat.

11. Verfahren nach einem oder mehreren der Ansprüche 7 bis 10, umfassend eine Pelletisierbehandlung vor der Wärmebehandlung.

12. Verfahren nach einem oder mehreren der Ansprüche 7 bis 11, worin die Wärmebehandlung bei einer Temperatur zwischen 300°C und 800°C ausgeführt wird.

13. Verfahren nach einem oder mehreren der Ansprüche 7 bis 12, worin die Wärmebehandlung in einer oxidierenden Umgebung ausgeführt wird.

14. Verfahren zur Oligomerisation von Alkenen, umfassend das Umsetzen eines oder mehrerer Alkene mit einem oder mit mehreren Zeolithen gemäß einem oder mehreren der Ansprüche 1 bis 6 und das Gewinnen von oligomerisierten Produkten hieraus.

15. Verfahren nach Anspruch 14, umfassend die Verwendung eines oder mehrerer Alkene mit bis zu 6 Kohlenstoffatomen als Einsatzmaterial zur Ausbildung von Oligomeren, insbesondere die Verwendung von Ethylen oder 1-Buten als Einsatzmaterial.

16. Verfahren nach Anspruch 14 oder 15, worin die Umsetzung bei einer Temperatur zwischen 150°C und 400°C und bei einem Druck von bis zu 100 bar ausgeführt wird, insbesondere bei einer Temperatur zwischen 200°C und 350°C und bei einem Druck zwischen 10 und 50 bar.

17. Zur Verwendung in einem Verfahren nach einem oder mehreren der Ansprüche 14 bis 16 geeignete Katalysatoren, umfassend einen Zeolithen nach einem oder mehreren der Ansprüche 1 bis 6 und ein Bindemittel.

18. Katalysatoren nach Anspruch 17, umfassend einen gemäß einem Verfahren nach einem oder mehreren der Ansprüche 7 bis 13 hergestellten Zeolithen.

19. Oligomerisierte Produkte, erhalten nach einem Verfahren nach einem oder mehreren der Ansprüche 14 bis 16.

## Revendications

1. Zéolites comprenant une fonction métal A et une fonction métal B, où la fonction A est constituée d'un groupement métal alcalin et la fonction B est constituée d'au moins un groupement de métal d'un métal du groupe VIb, VIIb ou VIII, zéolites qui possèdent un rapport atomique Si/Al d'au moins 3 et une valeur A/Aₒ (telle que définie dans la suite du présent mémoire) d'au plus 0,05, cependant que le rapport atomique métal alcalin/aluminium varie de 0,6 à 1,1, où Aₒ représente l'absorption de la bande OH, possédant son pic maximal dans la plage des fréquences comprises entre 3600 cm⁻¹ et 3650 cm⁻¹ dans le spectre infrarouge de l'échantillon de zéolite correspondante sous la forme H et où A représente l'absorbance de la bande OH au nombre d'ondes correspondant de la zéolite conforme à la présente invention.

2. Zéolites selon la revendication 1, caractérisées en ce que la fonction A comprend un groupement potassium.

3. Zéolites suivant la revendication 1 ou 2, caractérisées en ce que la fonction B comprend un groupement métal du groupe VIII, en particulier le nickel.

4. Zéolites suivant une ou plusieurs des revendications 1 à 3, caractérisées en ce que le rapport atomique Si/Al est compris entre 5 et 100.

5. Zéolites suivant une ou plusieurs des revendications 1 à 4, caractérisées en ce que les zéolites possèdent des structures cycliques contenant jusqu'à 12 atomes T, plus particulièrement du type MFI ou MEL.

6. Zéolites suivant une ou plusieurs des revendications 1 à 5, caractérisées en ce que le rapport atomique métal alcalin/aluminium est compris entre 0,65 et 1.

7. Procédé de préparation de zéolites suivant une ou plusieurs des revendications 1 à 6, caractérisé en ce que l'on soumet une zéolite possédant un rapport atomique Si/Al d'au moins 3 et un rapport atomique métal alcalin/aluminium compris entre 0,6 et 1,1, ensemble avec un composé de métal du groupe VIB, VIIB ou VIII, à des conditions d'échange à l'état solide, y compris un traitement thermique pour réduire l'indice A/Aₒ jusqu'à une valeur d'au plus 0,05, où Aₒ représente l'absorption de la bande OH, possédant son pic maximal dans la plage des fréquences comprises entre 3600 cm⁻¹ et 3650 cm⁻¹ dans le spectre infrarouge de l'échantillon de zéolite correspondante sous la forme H et où A représente l'absorbance de la bande OH au nombre d'ondes correspondant de la zéolite conforme à la présente invention.

8. Procédé suivant la revendication 7, caractérisé en ce que l'on soumet la zéolite à un traitement par un composé de métal du groupe VIII, plus particulièrement un composé de nickel.

9. Procédé suivant la revendication 7 ou 8, caractérisé en ce que l'on soumet la zéolite à un traitement par un composé de métal contenant un anion décomposable, plus particulièrement, un nitrate.

10. Procédé suivant la revendication 9, caractérisé en ce que l'on soumet la zéolite à un traitement par du nitrate de nickel nonahydraté.

11. Procédé suivant une ou plusieurs des revendications 7 à 10, caractérisé en ce que l'on procède à un traitement de granulation ou de pastillage avant de procéder au traitement thermique.

12. Procédé suivant une ou plusieurs des revendications 7 à 11, caractérisé en ce que l'on entreprend le traitement thermique à une température comprise entre 300°C et 800°C.

13. Procédé suivant une ou plusieurs des revendications 7 à 12, caractérisé en ce que l'on réalise le traitement thermique dans un environnement oxydant.

14. Procédé d'oligomérisation d'alcènes, caractérisé en ce que l'on fait réagir un ou plusieurs alcènes avec une ou plusieurs zéolites suivant une ou plusieurs des revendications 1 à 6 et on récupère les produits oligomérisés.

15. Procédé suivant la revendication 14, caractérisé en ce que l'on utilise un ou plusieurs alcènes possédant jusqu'à 6 atomes de carbone à titre de charge de départ pour produire des oligomères, plus particulièrement, en utilisant de l'éthylène ou du 1-butène comme charge de départ.

16. Procédé suivant la revendication 14 ou 15, caractérisé en ce que l'on entreprend la réaction à une température comprise entre 150°C et 400°C et sous une pression allant jusqu'à 100 bars, plus particulièrement, à une température comprise entre 200°C et 350°C et sous une pression comprise entre 10 et 50 bars.

17. Catalyseurs convenant à l'utilisation pour la mise en oeuvre d'un procédé suivant une ou plusieurs des revendications 14 à 16, caractérisés en ce qu'ils comprennent une zéolite telle que revendiquée dans une ou plusieurs des revendications 1 à 6 et un liant.

18. Catalyseurs suivant la revendication 17, caractérisés en ce qu'ils comprennent une zéolite préparée suivant un procédé tel que revendiqué dans une ou plusieurs des revendications 7 à 13.

19. Produits oligomérisés lorsqu'ils sont obtenus par mise en oeuvre d'un procédé tel que revendiqué dans une ou plusieurs des revendications 14 à 16.
